# EUROPEAN PATENT APPLICATION

(11) **EP 4 374 914 A1**
(43) Date of publication of application: **29.05.2024**
(21) Application number: 22845958.2
(22) Date of filing: 20.07.2022
(51) Int. Cl.: A61P 9/10, A61P 27/02, C12M 3/00, C12N 5/07, C12N 5/0735, C12N 5/10, A61K 35/30, A61L 27/38, A61L 27/40, C07K 14/78

(54) **METHOD FOR PRODUCING RETINAL TISSUE**

(30) Priority: 21.07.2021 JP 2021120128
(71) Applicant: Kyoto University, Kyoto-shi, Kyoto 606-8501 (JP)
(72) Inventor: EIRAKU, Mototsugu, Kyoto-shi, Kyoto 606-8501 (JP)
(74) Representative: Hoffmann Eitle
(86) International application number: PCT/JP2022/028243
(87) International publication number: WO 2023/003025

(57) **Abstract**

The present disclosure includes a method of producing a retinal tissue, comprising inducing differentiation of pluripotent stem cells into a retinal tissue on a surface of a culture substrate, wherein the culture substrate comprises a region A and a region B on the surface, the differentiation is induced is the region A, the region A has cell adhesiveness, and the region B is adjacent to at least a part of the region A and has cell adhesiveness lower than the cell adhesiveness of the region A; a retinal tissue produced by the method; and a composition comprising the retinal tissue.

## Description

### TECHNICAL FIELD

This application claims benefit of priority to Japanese Patent Application No. 2021-120128, the entire content of which is herein incorporated by reference.

The present disclosure includes a method of producing a retinal tissue, a retinal tissue produced by the method, and a composition comprising the retinal tissue.

### BACKGROUND

Culturing techniques of pluripotent stem cells have been actively studied for forming organs in test tubes for the purpose of transplantation and drug efficacy evaluation. The Serum-free Floating culture of Embryonic Body-like aggregates with quick reaggregation (SFEBq method) is a method to induce differentiation of pluripotent stem cells into cells of the central nervous system by culturing pluripotent stem cells in suspension culture for several days in a medium not containing components that inhibit neural differentiation, such as serum and transcription factors. The method has also been applied for forming a retinal tissue from pluripotent stem cells. The SFEBq method mimics the process of ontogenesis *in vitro* relying on self-assembly of cells, and can stably produce three-dimensional tissues with good reproducibility. Since the SFEBq method relies on the self-assembly of cells, however, the patterning process may vary and it is difficult to control morphology and size of tissues. Also, due to the thickness of the resulting cell mass, it is difficult for drugs to reach cells within the cell mass, and also difficult to observe such inside cells. Furthermore, the SFEBq method is not considered to be suited for automation. Therefore, there is a need for a suitable method for stable supply and automatic production of retinal tissues.

### SUMMARY OF THE INVENTION

### PROBLEMS TO BE SOLVED BY THE INVENTION

An object of the present disclosure is to provide a method of producing a retinal tissue. A further object of the present disclosure is to provide a retinal tissue produced by the method and a composition comprising the retinal tissue.

### SOLUTIONS TO THE PROBLEMS

In an aspect, the present disclosure relates to a method of producing a retinal tissue, comprising inducing differentiation of pluripotent stem cells into a retinal tissue on a surface of a culture substrate, wherein the culture substrate comprises a region A and a region B on the surface, the differentiation is induced is the region A, the region A has cell adhesiveness, and the region B is adjacent to at least a part of the region A and has cell adhesiveness lower than the cell adhesiveness of the region A.

In a further aspect, the present disclosure relates to a retinal tissue produced by the method of the present disclosure.

In a further aspect, the present disclosure relates to a composition comprising the retinal tissue of the present disclosure.

### EFFECTS OF THE INVENTION

The present disclosure provides a method for producing a retinal tissue, a retinal tissue produced by the method, and a composition comprising the retinal tissue.

### BRIEF DESCRIPTION OF THE DRAWINGS

Fig. 1 shows the experimental scheme for induction of retinal differentiation.
Fig. 2 shows the gene ontology (GO) analysis of patterned cells before BMP4 addition (Day 8).
Fig. 3 shows the GO analysis of non-patterned cells before BMP4 addition (Day 8).
Fig. 4 shows the time course of GFP (Rx::venus) expression in cells after induction of retinal differentiation with (top) or without (bottom) patterning culture.
Fig. 5 shows Chx10 (top) and GFP (bottom) expressions at Day 18 in patterned (Patterning) and non-patterned (All) cells. The results of BMP4-added cells (BMP +) and BMP4-free cells (BMP -) are shown.
Fig. 6 shows GFP (Rx::venus) and Chx10 expressions at Day 18 in cells after induction of retinal differentiation with patterning culture.
Fig. 7 shows GFP (Rx::venus), Recoverin, and Chx10 expressions at Day 58 in cells after induction of retinal differentiation with patterning culture.
Fig. 8 shows GFP (Rx::venus) and FOXG1 expressions at Day 18 in patterned BMP4-free cells.
Fig. 9 shows the efficiency of differentiation induction in cells after induction of retinal differentiation with patterning culture.
Fig. 10 shows GFP expressions at Day 20 of patterned cells in various sizes.
Fig. 11 shows Crx expression of the tissue at Day 40.
Fig. 12 shows the induction of retinal differentiation by patterning culture with KthES 11 cells. Expressions of Crx, Chx 10, and Tuj1 at Day 50 are shown.
Fig. 13 shows the induction of retinal differentiation by patterning culture with 201B7 cells (top) and 253G1 cells (bottom). Expressions of Crx and Chx10 at Day 34 are shown.
Fig. 14 shows GFP expression at Day 11 with (top) or without (bottom) LDN-193189 addition.
Fig. 15 shows Chx10 (top) and GFP (bottom) expressions in patterned (Patterning) and non-patterned (All) cells with or without LDN-193189 addition. The results at Day 18 are shown.
Fig. 16 shows GFP and Chx10 expressions in patterned (Patterning) and non-patterned (All) cells with or without LDN-193189 addition. The scale bar indicates 1 mm. The results at Day 18 are shown.
Fig. 17 shows the time course of retinal differentiation induction by patterning culture with an MPC polymer.

### DETAILED DESCRIPTION

Unless otherwise defined, the terms used herein are read as generally understood by those skilled in the technical fields such as organic chemistry, medical sciences, pharmaceutical sciences, molecular biology, and microbiology. Several terms used herein are defined as below. The definitions herein take precedence over the general understandings.

When a numerical value is accompanied with the term "about", the value is intended to represent any value in the range of -10% of the value to +10% of the value. A range defined with values of lower and upper limits covers all values from the lower limit to the upper limit, including the values of the both limits. When a range is accompanied with the term "about", the both limits are read as accompanied with the term. For example, "about 20 to 30" is read as "18 to 33".

The method of producing a retinal tissue of the present disclosure comprises inducing differentiation of pluripotent stem cells into a retinal tissue on a surface of a culture substrate, wherein the culture substrate comprises a region A and a region B on the surface, the differentiation is induced is the region A, the region A has cell adhesiveness, and the region B is adjacent to at least a part of the region A and has cell adhesiveness lower than the cell adhesiveness of the region A.

The region A is a region that can maintain pluripotent stem cells in adhesive culture. The region B is a region having cell adhesiveness lower than that of the region A so that, when pluripotent stem cells are seeded on the region A, the cells are prevented from extending from the region A to the region B adjacent to the region A during the induction of retinal tissue differentiation (i.e. the period until differentiation to a retinal tissue is confirmed). The cell adhesiveness can be compared by seeding pluripotent stem cells of an excess amount relative to an area (e.g. 5 × 10⁵ to 10 × 10⁵ cells/cm²) and comparing the percentage of the area to which the cells adhere after 24 hours culture at 37° C and 5% CO₂. The cell adhesiveness is typically evaluated after the medium is removed after the 24 hours culture and the cells are washed with a medium or buffer to remove unadhered cells.

The region A may be, for example, a region where, when pluripotent stem cells of an excess amount relative to its area (e.g. 5 × 10⁵ to 10 × 10⁵ cells/cm²) are seeded and cultured at 37°C and 5% CO₂ for 24 hours, the cells adhere to 70% or more, 80% or more, or 90% or more of the area after the 24 hours.

In some embodiments, the region B is a region having no cell adhesiveness. The region having no cell adhesiveness refers to a region that does not have sufficient cell adhesiveness to maintain pluripotent stem cells in adhesive culture. The region having no cell adhesiveness may be, for example, a region where, when pluripotent stem cells of an excess amount relative to its area (e.g. 5 × 10⁵ to 10 × 10⁵ cells/cm²) are seeded and cultured at 37°C and 5% CO₂ for 24 hours, the cells adhere to 30% or less, 20% or less, or 10% or less of the area after the 24 hours.

The culture substrate may be made of any material as long as it can have the region A and the region B on its surface. For example, the culture substrate may be made of an inorganic material such as metal, glass, or silicone, or an organic material such as plastic (e.g., polystyrene resin, polyethylene resin, polypropylene resin, ABS resin, nylon, acrylic resin, fluorine resin, polycarbonate resin, polyurethane resin, methylpentene resin, phenol resin, melamine resin, epoxy resin, vinyl chloride resin, polytetrafluoroethylene tetrafluoroethylene resin). In some embodiments, the culture substrate is made of glass or plastic, especially polystyrene resin.

The culture substrate may have any shape commonly used for cell culture. The culture substrate may be, for example, a culture vessel such as a petri dish, plate, bottle, chamber, or multi-well plate (e.g., 6, 12, 24, 48, 96, or 384 well plate), or may be a film or porous membrane. The culture substrate typically has the region A and the region B on the surface that is horizontal to the direction of gravity (on the bottom surface, when the culture substrate is a culture vessel).

The region A may be a region of the surface of a cell-adhesive culture substrate, or a region of the surface of a culture substrate coated with a cell-adhesive material to make the surface cell-adhesive or to enhance the cell-adhesiveness of the surface. Examples of cell-adhesive materials include positively charged polymers such as poly L-lysine and poly L-omithine; laminin; collagens such as type I collagen, type II collagen, type III collagen, type IV collagen, type V collagen, and type VII collagen; tenascin; fibrillin; fibronectin; vitronectin; elastin; entactin; proteoglycans composed of sulfated glucosaminoglycans such as chondroitin sulfate, heparan sulfate, keratan sulfate, and dermatan sulfate and core proteins; glucosaminoglycans such as chondroitin sulfate, heparan sulfate, keratan sulfate, dermatan sulfate, and hyaluronic acid; Synthemax^{®} (a vitronectin derivative) and Matrigel^{®}.

In some embodiments, the region A is coated with laminin. Laminin is a heterotrimeric molecule comprising three subunit chains, α, β, and γ. There are five types of α chains, α1-α5, three types of β chains, β1-β3, and three types of γ chains, γ1-γ3, and each laminin isoform is represented by the numbers indicating its constituent subunits (for example, laminin 111 is composed of α1 chain, β1 chain, and γ1 chain). Examples of laminins include laminin 111, laminin 121, laminin 211, laminin 213, laminin 222, laminin 311 (laminin 3A11), laminin 332 (laminin 3A32), laminin 321 (laminin 3A21), laminin 3B32, laminin 411, laminin 421, laminin 423, laminin 521, laminin 522, laminin 523, or fragments thereof. Examples of fragments of laminins include E8 fragments, which are fragments of integrin binding sites, such as laminin 211-E8, laminin 311-E8, laminin 411-E8, and laminin 511-E8. In some embodiments, laminin is laminin 511 or a fragment thereof. In further embodiments, laminin is the laminin 511-E8 fragment. For coating with laminin, a commercial product such as iMatrix-511 (Nippi. Inc.) may be used. iMatrix-511 (Nippi. Inc.) contains the laminin 511-E8 fragment.

The region B may be a region of the surface of a culture substrate having cell adhesiveness lower than that of the region A, or a region of the surface of a culture substrate coated with a material to make the region less cell-adhesive than the region A. In some embodiments, the region B is coated with a cell non-adhesive material. Examples of non-cell-adhesive materials include MPC (2-methacryloyloxyethyl phosphorylcholine) polymers; celluloses such as methyl cellulose, hydroxyethyl cellulose, hydroxypropyl cellulose, hydroxypropyl methyl cellulose, and sodium carboxymethyl cellulose; polyethylene oxide; carboxyvinyl polymers; polyvinylpyrrolidone; polyethylene glycol; polyamides such as polyacrylamide and poly-N-isopropylacrylamide; polysaccharides such as chitin, chitosan, hyaluronic acid, alginic acid, starch, pectin, carrageenan, guar gum, gum arabic, and dextran; albumin and their derivatives. In some embodiments, the non-cell-adhesive material is an MPC polymer.

The region A may have any shape, and for example, may have, a circular, oval, or polygonal (e.g., triangle, tetragonal, pentagonal, hexagonal, octagonal, decagonal, or dodecagonal) shape. In some embodiments, the region A has a circular shape. As used herein, a circular shape refers to a shape that is recognized as substantially circular in the art, including a perfect circle. The area of the region A may be, but is not limited to, 0.01 to 100 cm², 0.01 to 30 cm², 0.01 to 10 cm², 0.03 to 30 cm², 0.03 to 10 cm², or 0.1 to 10 cm². In some embodiments, the area of the region A is 0.1, 0.2, 0.3, 0.4, 0.5, 0.6, 0.7, 0.8, 0.9, or 1 cm² or more, and 10, 9, 8, 7, 6, 5, 4, 3, 2, or 1 cm² or less. For example, the area of the region A may be 0.5 to 10 cm², 0.7 to 10 cm², 1 to 10 cm², 0.5 to 5 cm², 0.7 to 5 cm², or 1 to 5 cm².

The region A may have a circular shape with a diameter of 0.1, 0.2, 0.3, 0.4, 0.5, 0.6, 0.7, 0.8, 0.9, or 1 cm or more, and 10, 9, 8, 7, 6, 5, 4, 3, 2, or 1 cm or less, or a polygonal shape having an equal area to the circular shape. In some embodiments, the region A has a circular shape with a diameter of 0.1 to 10 cm, 0.1 to 5 cm, 0.1 to 3 cm or 0.2 to 1 cm. In further embodiments, the region A has a circular shape with a diameter of 1 to 10 cm, 1 to 5 cm, or 1 to 3 cm.

At least a part of the region A is adjacent to the region B. In some embodiments, the entire outer edge of the region A is adjacent to the region B (i.e., the region A is surrounded by the region B). The distance between two regions A flanking the region B may be, but is not limited to, about 1 mm or more.

The regions A and B may be formed on the surface of a culture substrate by any of methods used for cell patterning. The regions A and B can be formed on the surface of a culture substrate by techniques such as soft lithography, photolithography, and 3D printing, for example.

The regions A and B may be formed by treating a portion of the surface of a culture substrate to make the treated and non-treated regions have different cell adhesive properties. For example, these regions may be formed by masking a portion of the surface of a culture substrate and coating the unmasked area with a cell-adhesive or non-cell-adhesive material. Alternatively, these regions may be formed by providing a layer of a cell-adhesive or non-cell-adhesive material on a culture substrate and treating a portion of the layer to alter the cell adhesiveness. The treated portion can be a portion where the surface of the culture substrate is exposed or a portion where the property of the non-cell-adhesive material or cell-adhesive material has been changed by the treatment.

For example, the regions A and B may be formed on a culture substrate by preparing a sheet with a hole of the shape and size of the region A using a 3D printed mold, covering the surface of the culture substrate with the sheet, coating the portion of the surface not covered with the sheet with a cell-adhesive material, and then removing the sheet. Alternatively, the regions A and B may be formed on a culture substrate by preparing a sheet of the shape and size of the region A, placing the sheet on the surface of the culture substrate, coating the portion of the surface not covered with the sheet with a non-cell-adhesive material, removing the sheet, and coating the portion of the surface covered with the sheet with a cell-adhesive material. The sheet may be prepared from a biocompatible material such as polydimethylsiloxane (PDMS), polyethylene glycol hydrogel, or agarose gel. The coating can be performed, for example, by bringing the culture substrate into contact with a solution of a cell-adhesive or non-cell-adhesive material and allowing it to react with the solution at 37°C or room temperature for the required time (e.g., 1 hour or more). The concentration of the cell-adhesive or non-cell-adhesive material can be appropriately determined by those skilled in the art, and, for example, for laminin, may be 0.1 to 1 µg/cm², 0.1 to 0.5 µg/cm², or about 0.25 µg/cm².

In the present disclosure, the culture substrate may have a convex portion (also called a pillar) on the surface and the convex portion may have the region A on its upper surface. The shape of the convex portion may be, but is not limited to, a circular cylinder or a square cylinder. As used herein, a circular cylinder refers to a column whose cross section is recognized as a substantially circular shape in the art, including a perfect circle. In some embodiments, the convex portion is in the form of a circular cylinder. The height of the convex portion may be, but is not limited to, 0.1 mm to 10 mm, 0.1 mm to 5 mm, 1 mm to 5 mm, or 3 mm to 5 mm, for example 4 mm. The material of the convex portion may be the same as or different from that of the culture substrate. In addition to those exemplified herein as materials for the culture substrate, examples of materials for the convex portion include polydimethylsiloxane (PDMS), polymethylmethacrylate (PMMA), polyethylene terephthalate (PET), polyamide (PA), and polymethylglutarimide (PMGI), polyvinyl alcohol (PVA), polyethylene glycol (PEG), polyethylene vinyl acetate (PEVA), and polyethylene oxide (PEO).

Pluripotent stem cells are cells that have pluripotency, which is an ability to differentiate into all types of cells that make up an adult body, and self-renewal capacity, which is an ability to maintain pluripotency after cell division. The pluripotent stem cells can be either newly established cells or cells of a cell line. The pluripotent stem cells include embryonic pluripotent stem cells (ES cells), embryonic germ cells (EG cells), and induced pluripotent stem cells (iPS cells). The species of pluripotent stem cells is preferably, but not limited to, mammalian (e.g., human, monkey, mouse, rat, hamster, guinea pig, dog, cat, pig, cattle, goat, horse, sheep, rabbit). In some embodiments, the pluripotent stem cells are rodent (e.g., mouse, rat, hamster, guinea pig) or primate (e.g., human, monkey), preferably primate. In further embodiments, the pluripotent stem cells are monkey or human pluripotent stem cells; monkey or human ES or iPS cells; or human iPS cells.

ES cells are pluripotent stem cells that are derived from early embryos and can be established from the inner cell mass of blastocysts or from the epiblast of early embryos after implantation. ES cells may be cells of a cell line, such as Kh-ES-1 cells, Kh-ES-2 cells, Kh-ES-13 cells, and KthES11 cells (available from the Institute for Virus and Regenerative Medicine, Kyoto University). In some embodiments, ES cells are not ES cells that have been established by any of methods involving the destruction of human embryos.

iPS cells are cells that are derived from cells other than pluripotent stem cells, such as somatic cells, and may be produced by any method, e.g., a method described in WO2007/069666, WO2009/006930, WO2009/006997, WO2009/007852, WO2008/118820, Cell 126(4): 663-676 (2006), Cell 131(5): 861-872 (2007), Science 318(5858): 1917-1920 (2007), Nature Biotechnology 26(1): 101-106 (2008), Cell Stem Cell 3(5): 568-574 (2008), Cell Stem Cell 4(5): 381-384 (2009), Nature 454: 646-650 (2008), Cell 136(3): 411-419 (2009), Nature Biotechnology 26: 1269-1275 (2008), Cell Stem Cell 3: 475-479 (2008), Nature Cell Biology 11: 197-203 (2009), Cell 133(2): 250-264 (2008), Science, 2013, 341 pp. 651-654, Stem Cells 31: 458-466 (2013). iPS cells may also be cells of a cell line, such as 201B7 cells, 201B7-Ff cells, 253G1 cells, 253G4 cells, 1201C1 cells, 1205D1 cells, 1210 B2 cells, 1231A3 cells, Ff-I01 cells, Ff-I14 cells, and QHJI01 cells (available from Kyoto University or iPS Academia Japan, Inc.).

In some embodiments, iPS cells are prepared by introducing into somatic cells, such as fibroblasts, skin cells, or hematopoietic cells (e.g., peripheral blood mononuclear cells or T cells, or cord blood-derived cells), a combination of reprogramming factors selected from Oct3/4, Sox2, Klf4, Myc (c-Myc, N-Myc, L-Myc), Glisl, Nanog, Sall4, lin28 and Esrrb. Examples of preferred combinations of reprogramming factors include (1) Oct3/4, Sox2, Klf4, and Myc (c-Myc or L-Myc), and (2) Oct3/4, Sox2, Klf4, Lin28, and L-Myc.

The pluripotent stem cells are generally seeded on a culture substrate at a cell number so that the cells occupy at least 70% of the region A. The cells may be seeded only in the region A or on the entire region of the culture substrate including the region A and the region B. For example, when the region B is a non-cell-adhesive region, the cells can be seeded on the entire region of the culture substrate. For example, the pluripotent stem cells may be seeded at 5 × 10⁵ to 10 × 10⁵ cells/cm². Typically, pluripotent stem cells are collected and dispersed with a cell dispersing solution containing an enzyme (e.g., trypsin, collagenase, hyaluronidase, elastase, pronase, DNase, or papain) and/or a chelating agent (e.g., ethylenediaminetetraacetic acid (EDTA)), and suspended in a desired medium. Then, the resulting cell suspension is added onto the culture substrate. As the cell dispersing solution, a commercial product such as TrypLE^{™} Select (Thermo Fisher Scientific) or TrypLE^{™} Express (Thermo Fisher Scientific) may be used, for example.

Differentiation induction of pluripotent stem cells into a retinal tissue is performed by adhesion culture on the culture substrate described above. The differentiation induction of pluripotent stem cells into a retinal tissue may be started after pluripotent stem cells seeded on a culture substrate are cultured for a certain period of time in a medium containing a factor for maintaining undifferentiated state. In the present disclosure, the time when the culturing in a medium that does not contain a factor for maintaining undifferentiated state is started is defined as the time when the differentiation induction into a retinal tissue is started.

In the present disclosure, the differentiation induction of pluripotent stem cells into a retinal tissue may be performed by any method available to those skilled in the art. In some embodiments, the inducing differentiation of pluripotent stem cells into a retinal tissue comprises culturing pluripotent stem cells in a medium containing a BMP signaling activator. In further embodiments, the inducing differentiation of pluripotent stem cells into a retinal tissue comprises the following steps:
(1) culturing pluripotent stem cells in a medium containing a BMP signaling inhibitor; and
(2) culturing the cells obtained by the step (1) in a medium containing a BMP signaling activator.

The culturing the cells in the presence of a BMP signaling inhibitor before adding a BMP signaling activator can increase the efficiency of differentiation into a retinal tissue. In other words, the present disclosure provides, in an aspect, a method of producing a retinal tissue comprising the steps (1) and (2) as described above. In this aspect, the culturing in the steps (1) and (2) is preferably performed by adhesion culture.

The medium used in the method of the present disclosure can be prepared by using a medium commonly used for culturing animal cells as a basal medium. For example, the basal medium may be IMDM, DMEM, F-12, DMEM/F12, IMDM/F12, BME, BGJb, CMRL 1066, Glasgow MEM (GMEM), Improved MEM Zinc Option, Medium 199, Eagle MEM, alpha MEM, Ham's medium, RPMI 1640, Fischer's medium, or a mixture thereof.

The medium may contain one or more components selected from serum, serum substitutes, growth factors, factors for maintaining undifferentiated state, proteins (e.g., cytokines, insulin), fatty acids, lipids, vitamins, amino acids (e.g., nonessential amino acids, retinoids, glutamine, taurine), antioxidants, 2-mercaptoethanol, 1-thioglycerol, antibiotics, buffers, and inorganic salts, which are added to a basal medium as needed. Examples of serum substitutes include, for example, albumin such as bovine serum albumin (BSA), transferrin, fatty acids, collagen precursors, trace elements, 2-mercaptoethanol, 1-thioglycerol, and their equivalents, and mixtures thereof. The medium may contain a commercially available serum substitute such as KnockOut^{™} Serum Replacement (Thermo Fisher Scientific), Chemically Defined Lipid Concentrate (Thermo Fisher Scientific), GlutaMAX^{™} Supplement (Thermo Fisher Scientific), Ham's F-12 Nutrient Mix, GlutaMAX^{™} Supplement (Thermo Fisher Scientific), B27 Supplement (Thermo Fisher Scientific), N2 Supplement (Thermo Fisher Scientific) or ITS Supplement (Thermo Fisher Scientific). The medium may be a commercial product that contains any of the above components in a basal medium, such as Ham's F-12 Nutrient Mix, GlutaMAX^{™} Supplement (Thermo Fisher Scientific), DMEM/F-12, GlutaMAXTM supplement (Thermo Fisher Scientific).

The medium is preferably a serum-free medium. The serum-free medium refers to a medium not containing unmodified or unpurified serum. A medium containing a purified blood-derived component or animal tissue-derived component is also included in the serum-free medium as long as it does not contain unmodified or unpurified serum. The serum-free medium may contain a serum substitute.

The differentiation induction is preferably performed in the absence of feeder cells (also described as under the feeder-free condition). The differentiation induction is also preferably performed under the xeno-free condition. In the present disclosure, the term xeno-free refers to being free of components that are derived from a species different from the species of the cells to be cultured.

Examples of factors for maintaining undifferentiated state include FGF signaling activators, TGFβ family signaling activators, and insulin. Examples of FGF signaling activators include FGF (e.g., bFGF, FGF4, FGF8). Examples of TGFβ family signaling activators include TGFβ signaling activators and Nodal/Activin signaling activators. Examples of TGFβ signaling activators include TGFβ1 and TGFβ2. Examples of Nodal/Activin signaling activators include Nodal, ActivinA, and ActivinB. For human pluripotent stem cells, factors for maintaining undifferentiated state preferably include bFGF. The concentration of a factor for maintaining undifferentiated state can be appropriately determined by a person skilled in the art, as long as it can maintain undifferentiated state of pluripotent stem cells. For example, when bFGF is used to culture human pluripotent stem cells, the concentration of bFGF may be 4 ng to 500 ng/mL, 10 ng to 200 ng/mL, or 30 ng to 150 ng/mL. A medium containing a factor for maintaining undifferentiated state may be a medium such as StemFit^{®} AK02N (Ajinomoto Co., Inc.), StemFit^{®} AK03N (Ajinomoto Co., Inc.), S-medium (DS Pharma Biomedical Co.), StemPro^{™} (Thermo Fisher Scientific), mTeSR1^{™} (STEMCELL Technologies), mTeSR2^{™} (STEMCELL Technologies), TeSR^{™}-E8^{™} (STEMCELL Technologies), or hESF9 (Proc. Natl. Acad. Sci. USA. 2008 Sep 9;105(36):13409-14). In some embodiments, the medium containing a factor for maintaining undifferentiated state is StemFit^{®} AK02N (Ajinomoto Co., Inc.).

The period of culturing in a medium containing a factor for maintaining undifferentiated state may be, but is not limited to, 1 to 10 days, 1 to 9 days, 1 to 8 days, 1 to 7 days, 1 to 6 days, 1 to 5 days, 1 to 4 days, 1 to 3 days, or 2 to 3 days.

The medium containing a factor for maintaining undifferentiated state may additionally contain a ROCK inhibitor. Examples of ROCK inhibitors include Y-27632, Fasudil (HA1077), and H-1152. The concentration of the ROCK inhibitor is appropriately determined according to the inhibitor to be used, and may be 1 to 100 µM, 5 to 50 µM, or about 10 µM, or a concentration providing a ROCK inhibitory activity equivalent to that of Y-27632 at the concentration as mentioned above. The period of culturing in a medium further containing a ROCK inhibitor may be a part or the whole of the period of culturing in a medium containing a factor for maintaining undifferentiated state. For example, the cells may be cultured in a medium containing a factor for maintaining undifferentiated state and a ROCK inhibitor for 1 to 2 days, and then cultured in a medium containing a factor for maintaining undifferentiated state but not a ROCK inhibitor for 1 to 2 days.

BMP (bone morphogenetic protein) includes, for example, BMP2, BMP4, BMP7, and BMP12 (GDF7). The BMP signaling activator and inhibitor may be an activator and an inhibitor of one or more of these BMPs, respectively.

A BMP signaling inhibitor is a substance that inhibits signaling by BMP, and includes substances that act on BMP or its receptor, substances that suppress gene expression of BMP or its receptor, and substances that inhibit binding between BMP and its receptor. Examples of BMP signaling inhibitors include LDN-193189 4-[6-(4-Piperazin-1-ylphenyl)pyrazolo[1,5-a]pyrimidin-3-yl]quinoline), Dorsomorphin (6-[4-[2-(1-Piperidinyl)ethoxy]phenyl]-3-(4-pyridinyl)-pyrazolo[1,5-a]pyrimidine), and DMH1 (4-(6-(4-isopropoxyphenyl)pyrazolo[1,5-a]pyrimidin-3-yl)quinoline). In some embodiments, the BMP signaling inhibitor is LDN-193189. The concentration of a BMP signaling inhibitor is determined according to the inhibitor to be used, and may be 1 to 1000 nM, 10 to 500 nM, 30 to 300 nM, or about 100 nM, or a concentration providing a BMP inhibitory activity equivalent to that of LDN-193189 at the concentration as mentioned above.

The period of culturing in a medium containing a BMP signaling inhibitor may be, but is not limited, 1 to 10 days, 2 to 9 days, 3 to 7 days, 4 to 6 days, or about 5 days.

A BMP signaling activator is a substance that activates signaling by BMP, and includes substances that act on BMP or its receptor, substances that enhance gene expression of BMP or its receptor, and substances that promote binding between BMP and its receptor. Examples of BMP signaling activators include BMP2, BMP4, BMP7, BMP12 (GDF7) and fragments thereof, and anti-BMP receptor antibodies. In some embodiments, the BMP signaling activator is BMP4. The concentration of a BMP signaling activator is determined according to the activator to be used, and may be 0.01 to 1000 nM, 0.1 to 100 nM, 1 to 10 nM, 1 to 3 nM, or about 1.5 nM, or a concentration providing a BMP activity equivalent to that of BMP4 at the concentration as mentioned above.

The cells are cultured in a medium containing a BMP signaling activator for a period necessary for differentiation into a retinal tissue. The period of culturing may be, but is not limited to, 1 to 30 days, 2 to 20 days, 3 to 15 days, 4 to 12 days, or 5 to 10 days (e.g., 5, 6, 7, 8, 9, or 10 days). The concentration of a BMP signaling activator may be constant or varied during the culturing period. For example, the concentration of a BMP signaling activator may be decreased stepwise at a rate of 40 to 60% reduction per 2 to 4 days. For example, the concentration of a BMP signaling activator in a medium may be decreased stepwise by replacing a portion (e.g., half) of the medium containing the BMP signaling activator with a medium not containing the BMP signaling activator every 2, 3, or 4 days after the start of culturing in the medium containing the BMP signaling activator.

In some embodiments, the medium containing a BMP signaling inhibitor or BMP signaling activator is prepared from a 1:1 mixture of F-12 medium and IMDM medium containing KnockOut^{™} Serum Replacement (Thermo Fisher Scientific) (e.g., 0.5% to 30%, 1% to % to 20%, or 10%), Chemically Defined Lipid Concentrate (Thermo Fisher Scientific), BSA, and 1-thioglycerol.

After the culturing in a medium containing a BMP signaling activator, the medium may be replaced with a medium containing no BMP signaling activator to continue the culturing. This period of culturing may be, but is not limited to, 1 to 100 days, 10 to 90 days, 20 to 80 days, 30 to 70 days, 40 to 60 days, or about 50 days.

The cells may be cultured in a medium containing a Wnt signaling inhibitor during a part or the whole of the period of culturing in a medium containing a BMP signaling activator. A Wnt signaling inhibitor is a substance that inhibits signaling by Wnt, and includes substances that act on Wnt or its receptor, substances that suppress gene expression of Wnt or its receptor, and substances that inhibit binding between Wnt and its receptor. Examples of Wnt signaling inhibitors include CKI-7 (N-(2-Aminoethyl)-5-chloro-8-isoquinolinesulfonamide, D4476 (4-(4-(2,3-Dihydrobenzo[1,4]dioxin-6-yl)-5-pyridin-2-yl-1H-imidazol-2-yl)benzamide), IWR-1-endo (IWR1e) (4-[(3aR,4S,7R,7aS)-1,3,3a,4,7,7a-Hexahydro-1,3-dioxo-4,7-methano-2H-isoindol-2-yl]-N-8-quinolinylbenzamide) and IWP-2 (N-(6-methyl-2-benzothiazolyl)-2-[(3,4,6,7-tetrahydro-4-oxo-3 -phenylthieno [3,2-d]pyrimidin-2-yl)thio]-acetamide. The concentration of a Wnt signaling inhibitor is determined according to the inhibitor to be used, and may be 0.1 to 100 µM, 0.3 to 30 µM, or 1 µM to 10 µM.

During the culturing period, the medium may be replaced as needed. For example, a part or the whole of the medium may be replaced every 1 to 4 days. To start culturing in a medium containing a specific component, the whole of the medium may be replaced with a medium containing the component at a desired concentration, or a part of the medium may be replaced to achieve the desired final concentration of the component (e.g., half of the medium may be replaced with a medium at twice the final concentration). As described for the BMP signaling activator, the concentration of the component may be constant or varied during the culturing period in a medium containing the specific component.

In some embodiments, the inducing differentiation of pluripotent stem cells into a retinal tissue comprises
(1) culturing pluripotent stem cells in a medium containing a BMP signaling inhibitor for 4 to 6 days, and
(2) culturing the cells obtained by the step (1) in a medium containing a BMP signaling activator for 5 to 10 days.

In further embodiments, the inducing differentiation of pluripotent stem cells into a retinal tissue comprises
(1) culturing pluripotent stem cells in a medium containing a BMP signaling inhibitor for 4 to 6 days, and
(2) culturing the cells obtained by the step (1) in a medium containing a BMP signaling activator for 5 to 10 days,
and further comprises
prior to the step (1), culturing pluripotent stem cells in a medium containing a factor for maintaining undifferentiated state and a ROCK inhibitor for 1 to 2 days, and
culturing the cells obtained by the step (2) in a medium containing no BMP signaling activator for 1 to 100 days.

In further embodiments, the inducing differentiation of pluripotent stem cells into a retinal tissue comprises
(1) culturing pluripotent stem cells in a medium containing a BMP signaling inhibitor for 4 to 6 days, and
(2) culturing the cells obtained by the step (1) in a medium containing a BMP signaling activator for 5 to 10 days,
and further comprises
prior to the step (1), culturing pluripotent stem cells in a medium containing a factor for maintaining undifferentiated state and a ROCK inhibitor for 1 to 2 days, and then in a medium containing a factor for maintaining undifferentiated state but containing no ROCK inhibitor for 1 to 2 days, and
culturing the cells obtained by the step (2) in a medium containing no BMP signaling activator for 1 to 100 days.

Culture conditions such as culture temperature and CO₂ concentration can be appropriately determined. The culture temperature may be, for example, 30°C to 40°C or about 37°C. The CO₂ concentration may be, for example, 1% to 10% or about 5%.

Differentiation into a retinal tissue can be confirmed by detecting expression of a cell marker in cells of the tissue. The expression of a marker may be confirmed, for example, on any of Days 10 to 100 of differentiation induction (e.g., Day 10, 11, 12, 13, 14, 15, 16, 17, 18, 19, 20, 21, 22, 23, 24, 25, 26, 27, 28, 29, 30, 40, 50, 60, 70, 80, 90, or 100), or later. In some embodiments, the expression of a marker is confirmed on any of Days 18 to 22 of differentiation induction.

The retinal tissue in a living body has a layered structure and contains photoreceptor cells, horizontal cells, bipolar cells, amacrine cells, retinal ganglion cells, retinal pigment epithelial cells, Mueller cells, and their progenitor cells. As used herein, the cells that make up the retina are referred to as "retinal cells" and each layer that makes up the retina is referred to as a "retinal layer". Retinal layers in a mature retinal tissue include a retinal pigment epithelial layer, photoreceptor layer, outer limiting membrane, outer nuclear layer, outer plexiform layer, inner nuclear layer, inner plexiform layer, ganglion cell layer, nerve fiber layer and inner limiting membrane. Retinal tissues in a developmental stage before becoming mature retinal tissues may include a neuroblastic layer.

As used herein, the term "retinal progenitor cell" refers to a progenitor cell that can differentiate into one or more types of mature retinal cells selected from photoreceptor cell, horizontal cell, bipolar cell, amacrine cell, retinal ganglion cell, retinal pigment epithelial cell, and Müller cell. As used herein, the term "neural retinal progenitor cell" refers to a progenitor cell that can differentiate into one or more types of mature retinal cells selected from photoreceptor cell, horizontal cell, bipolar cell, amacrine cell, and retinal ganglion cell. Typically, a neural retinal progenitor cell does not differentiate into a retinal pigment epithelial cell.

As used herein, the term "retinal layer-specific neuronal cell" refers to a neuronal cell (neuron) that constitutes and is specific to a retinal layer. Examples of retinal layer-specific neuronal cells include photoreceptor cell (including rod cell and cone cell), horizontal cell, bipolar cell, amacrine cell, and retinal ganglion cell.

Examples of retinal cell markers include Rx (also called Rax), Pax6, and Chx10, which are expressed in retinal progenitor cells; Nkx2.1, which is expressed in progenitor cells of hypothalamic neurons but not in retinal progenitor cells; Sox1, which is expressed in the hypothalamic neuroepithelium but not in the retina; and Crx and Blimp1, which are expressed in photoreceptor progenitor cells. Examples of markers of retinal layer-specific neuronal cells include Chx10, PKCα, and L7, which are expressed in bipolar cells; Tuj1 and Brn3, which are expressed in retinal ganglion cells, Calretinin, which is expressed in amacrine cells; Calbindin, which is expressed in horizontal cells, Rhodopsin and Recoverin, which are expressed in mature photoreceptor cells; Nrl and Rhodopsin, which are expressed in rod cells; Rxr-gamma and S-Opsin, which are expressed in cone cells; and RPE65 and Mitf, whcih are expressed in retinal pigment epithelial cells. Other cell markers used in the examples can also be used.

In some embodiments, the retinal tissue produced by the method of the present disclosure contains Chx10-positive cells or Chx10-positive and Rx-positive cells at a percentage of 50% or more, 60% or more, 70% or more, 80% or more, 85% or more, 90% or more, or 95% or more. The percentage of the marker-positive cells may be the percentage at Day 10, 11, 12, 13, 14, 15, 16, 17, 18, 19, 20, 21, 22, 23, 24, 25, 26, 27, 28, 29, or 30 of differentiation induction or later. In some embodiments, the retinal tissue contains Chx10-positive and Rx-positive cells at a percentage of 70% or more, 80% or more, 85% or more, 90% or more, or 95% or more at any of Days 18 to 22 (e.g., Day 20) of differentiation induction. In further embodiments, the retinal tissue contains Chx10-positive and Rx-positive cells at a percentage of 90% or more or 95% or more at any of Days 18 to 22 (e.g., Day 20) of differentiation induction.

In some embodiments, the retinal tissue produced by the method of the present disclosure contains retinal cells at a percentage of 50% or more, 60% or more, 70% or more, 80% or more, 85% or more, 90% or more, or 95% or more. The percentage of the cells may be the percentage at any of Days 20 to 100 (e.g., Day 20, 21, 22, 23, 24, 25, 26, 27, 28, 29, 30, 40, 50, 60, 70, 80, 90, or 100) of differentiation induction or later. The retinal tissue preferably has a layered structure of cells.

The retinal tissue can be collected from the culture substrate in a usual manner. For example, the retinal tissue may be collected with an instrument such as a pair of tweezers. Alternatively, when the culture substrate is coated with a stimulus-responsive polymer (e.g., a temperature-responsive polymer or light-responsive polymer), the retinal tissue can be collected by applying the corresponding stimulus. For example, when a temperature-responsive polymer that reversibly changes its property from cell-adhesive (hydrophobic) to non-cell-adhesive (hydrophilic) at a certain temperature is used, the retinal tissue can be collected by placing the culture substrate at the temperature.

The collected retinal tissue may be maintained in a usual culture vessel or transferred to a suitable storage container for cryopreservation.

The shape and size of the retinal tissue depends on the shape and size of the region A. The area of the retinal tissue may be 0.01 to 100 cm², 0.03 to 30 cm², or 0.1 to 10 cm². In some embodiments, the area of the retinal tissue is 0.1, 0.2, 0.3, 0.4, 0.5, 0.6, 0.7, 0.8, 0.9, or 1 cm² or more, and 10, 9, 8, 7, 6, 5, 4, 3, 2, or 1 cm² or less. For example, the area of the retinal tissue may be 0.5 to 10 cm², 0.7 to 10 cm², 1 to 10 cm², 0.5 to 5 cm², 0.7 to 5 cm², or 1-5 cm². The retinal tissue may has a circular shape with a diameter of 0.1, 0.2, 0.3, 0.4, 0.5, 0.6, 0.7, 0.8, 0.9, or 1 cm or more, and 10, 9, 8, 7, 6, 5, 4, 3, 2, or 1 cm or less, or a polygonal shape having an equal area to the circular shape. In some embodiments, the retinal tissue has a circular shape with a diameter of 0.1 to 10 cm, 0.1 to 5 cm, 0.1 to 3 cm or 0.2 to 1 cm. In further embodiments, the retinal tissue has a circular shape with a diameter of 1 to 10 cm, 1 to 5 cm, or 1 to 3 cm. As used herein, a retinal tissue having a circular or polygonal shape refers to a retinal tissue that has a similar shape to the retinal tissue produced by the method of the present disclosure by using a culture substrate having a region A of the circular or polygonal shape. As used herein, the area and diameter of the retinal tissue refer to, respectively, the area within the outer periphery of the retinal tissue and the length of the longest straight line among the straight lines connecting any two points in the outer periphery, as measured based on an image taken by using a stereomicroscope. The retinal tissue may has a thickness of 10, 20, 30, 40, 50, 60, 70, 80, 90, or 100 µm or more, and 500, 400, 300, 290, 280, 270, 260, 250, 240, 230, 220, 210 or 220 µm or less. In some embodiments, the retinal tissue has a thickness of 50 µm to 300 µm. The retinal tissue need not have the same thickness throughout the tissue. When a retinal tissue is defined with a range of thickness, the retinal tissue may have a thickness within that range at any portion of the retinal tissue. The retinal tissue obtained by the method of the present disclosure usually contains about 30 cells or less in a direction perpendicular to the culture substrate, or has a thickness of 500 µm or less, and can also be referred to as a sheet-like retinal tissue or a retinal sheet. Thus, in one aspect, the present disclosure provides a sheet-like pluripotent stem cell-derived retinal tissue.

The retinal tissue produced by the method of the present disclosure can be used as a transplant material to treat a disease, especially a disease associated with or caused by a disorder of a retinal tissue or retinal cell. Therefore, the present disclosure provides a composition comprising the retinal tissue of the present disclosure for use in the treatment of a disease, and a method of treating a disease comprising implanting the retinal tissue of the present disclosure into a subject.

Examples of diseases associated with or caused by a disorder of a retinal tissue or retinal cell include retinal denaturation, retinitis pigmentosa, age-related macular degeneration, organic mercury poisoning, chloroquine retinopathy, glaucoma, diabetic retinopathy, and retinopathy of newborn babies. In some embodiments, the disease is retinitis pigmentosa.

When used for transplantation, the retinal tissue is preferably derived from pluripotent stem cells that are highly histocompatible with the subject (i.e., recipient) to whom the retinal tissue is transplanted. In some embodiments, the retinal tissue is derived from pluripotent stem cells that have a part or all of the histocompatibility antigens (e.g., HLA types) of the subject, or pluripotent stem cells established from cells of the subject.

The retinal tissue may be used for the treatment in its shape and size at the end of differentiation induction, or may be cut to an appropriate size with tweezers or other means in such a way that its layered structure is maintained. The composition comprising the retinal tissue of the present disclosure may comprise a pharmaceutically acceptable carrier in addition to the retinal tissue. The pharmaceutically acceptable carrier may be a physiological aqueous solvent such as saline, buffer solution, or serum-free medium. The composition may further comprises an additive such as a preservative, stabilizer, reducing agent, and isotonic agent. The composition comprising the retinal tissue of the present disclosure may be cryopreserved, and in this case, the composition may comprise a suitable cryoprotectant.

The retinal tissue produced by the method of the present disclosure may also be used for evaluating toxicity or efficacy of an agent on retinal tissues. In some embodiments, the retinal tissue of the present disclosure is used for evaluating toxicity or efficacy of a therapeutic agent for a disease associated with or caused by a disorder of a retinal tissue or retinal cell. In this case, the retinal tissue is preferably produced from pluripotent stem cells established from a subject suffering from the disease.

The retinal tissue produced by the method of the present disclosure has a structure suitable for transplantation or evaluation of toxicity or efficacy of an agent, and is to be used in such a way that its layered structure is maintained. In some cases, however, the retinal tissue may be dispersed (e.g., by ordinary means such as proteolytic enzymes or pipetting) to prepare a cell suspension, and the cell suspension may then be administered to a subject. This way of using the retinal tissue is also within the scope of this disclosure.

Exemplary embodiments of the present disclosure are shown below.
1. A method of producing a retinal tissue, comprising inducing differentiation of pluripotent stem cells into a retinal tissue on a surface of a culture substrate, wherein the culture substrate comprises a region A and a region B on the surface, the differentiation is induced is the region A, the region A has cell adhesiveness, and the region B is adjacent to at least a part of the region A and has cell adhesiveness lower than the cell adhesiveness of the region A.
2. The method according to item 1, wherein the region A is coated with a cell-adhesive material.
3. The method according to item 2, wherein the cell-adhesive material is laminin.
4. The method according to any one of items 1 to 3, wherein the region A is surrounded by the region B.
5. The method according to any one of items 1 to 4, wherein the region A has an area of 0.03 to 10 cm².
6. The method according to any one of items 1 to 5, wherein the region A has a circular shape with a diameter of 0.1 to 3 cm.
7. The method according to any one of items 1 to 6, wherein the region B is coated with a non-cell-adhesive material.
8. The method according to item 7, wherein the non-cell-adhesive material is an MPC polymer.
9. The method according to any one of items 1 to 8, wherein the inducing differentiation of pluripotent stem cells into a retinal tissue comprises culturing pluripotent stem cells in a medium containing a BMP signaling activator.
10. The method according to any one of items 1 to 9, wherein the inducing differentiation of pluripotent stem cells into a retinal tissue comprises the following steps:
   (1) culturing pluripotent stem cells in a medium containing a BMP signaling inhibitor; and
   (2) culturing the cells obtained by the step (1) in a medium containing a BMP signaling activator.
11. The method according to item 9 or 10, wherein the cells are cultured in the medium containing a BMP signaling activator for 5 to 10 days.
12. The method according to any one of items 9 to 11, wherein the BMP signaling activator is BMP4.
13. The method according to any one of items 10 to 12, wherein the cells are cultured in the medium containing a BMP signaling inhibitor for 4 to 6 days.
14. The method according to any one of items 10 to 13, wherein the BMP signaling inhibitor is LDN-193189.
15. The method according to any one of items 1 to 14, wherein the pluripotent stem cells are human iPS cells or ES cells.
16. The method according to any one of items 1 to 15, wherein the method comprises, prior to the inducing differentiation of pluripotent stem cells into a retinal tissue, culturing pluripotent stem cells in a medium containing a factor for maintaining undifferentiated state.
17. The method according to item 16, wherein the medium containing a factor for maintaining undifferentiated state further comprises a ROCK inhibitor.
18. A method of producing a retinal tissue, comprising the following steps:
   (1) culturing pluripotent stem cells in a medium containing a BMP signaling inhibitor; and
   (2) culturing the cells obtained by the step (1) in a medium containing a BMP signaling activator.
19. The method according to item 18, wherein the cells are cultured for 5 to 10 days in the medium containing a BMP signaling activator.
20. The method according to item 18 or 19, wherein the BMP signaling activator is BMP4.
21. The method according to any one of items 18 to 20, wherein the cells are cultured in the medium containing a BMP signaling inhibitor for 4 to 6 days.
22. The method according to any one of items 18 to 21, wherein the BMP signaling inhibitor is LDN-193189.
23. The method according to any one of items 18 to 22, wherein the pluripotent stem cells are human iPS cells or ES cells.
24. The method according to any one of items 18 to 23, wherein the method comprises, prior to the step (1), culturing pluripotent stem cells in a medium containing a factor for maintaining undifferentiated state.
25. The method according to item 24, wherein the medium containing a factor for maintaining undifferentiated state further comprises a ROCK inhibitor.
26. A retinal tissue produced by the method according to any one of items 1 to 17.
27. A retinal tissue produced by the method according to any one of items 18 to 25.
28. A sheet-like pluripotent stem cell-derived retinal tissue having an area of 0.5 to 10 cm².
29. A sheet-like pluripotent stem cell-derived retinal tissue having a circular shape with a diameter of 1 to 10 cm.
30. A composition comprising the retinal tissue according to any one of items 26 to 29.
31. The composition according to item 30, wherein the composition is for use in the treatment of a disease.
32. The composition according to item 31, wherein the disease is a disease associated with or caused by a disorder of a retinal tissue or retinal cell.
33. The composition according to item 31 or 32, wherein the disease is retinitis pigmentosa.
34. The composition according to item 30, wherein the composition is for use in evaluating toxicity or efficacy of an agent.
35. A method for treating a disease associated with or caused by a disorder of a retinal tissue or retinal cell, comprising implanting the retinal tissue according to any one of items 26 to 29 to a subject in need thereof.
36. The retinal tissue according to any one of items 26 to 29 for use in the treatment of a disease associated with or caused by a disorder of a retinal tissue or retinal cell.
37. Use of the retinal tissue according to any one of items 26 to 29 for the manufacture of a medicament for treating a disease associated with or caused by a disorder of a retinal tissue or retinal cell.

The invention will be further illustrated by the following examples, but should not limited in any way to these examples.

### EXAMPLES

### I. Patterning culture

### I-1. Preparation of PDMS sheet

1. A columnar mold having a shape intended for a cell-adhesive region at the top was prepared by a 3-D printer (Formlab 3).
2. PDMS (polydimethylsiloxane) (SILPOT 184 W/C, Dow Coming Toray Co. Ltd.) was mixed with a curing agent (10: 1) and degassed under vacuum.
3. After the mold prepared by the 3D printer was placed on the surface of a petri dish, PDMS was poured into the dish and cured at 80°C for 2 hours. Then, the prepared PDMS sheet was removed from the mold and unnecessary portions of the sheet were cut away to obtain an intended PDMS sheet.

### I-2. Differentiation induction

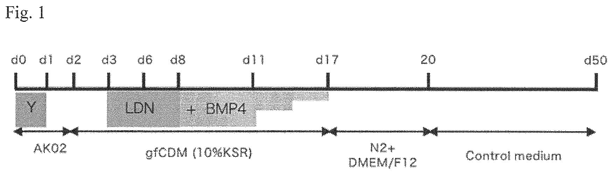

Differentiation into the retina was induced according to the following procedures. Fig. 1 shows the experimental scheme.

### <Day 0: Preparation of dish>

### Procedures

1. The PDMS sheet thus prepared was disinfected with 70% ethanol and placed on a dish (µ-Dish 35 mm, High, 81156, ibidi).
2. After the dish lid was removed, the dish was incubated at 60°C for 5 to 10 minutes in a heat block. The dish was dried in a dryer set at 60°C when it was difficult to be dried.
3. Seven µL of iMatrix-511 (Nippi. Inc.) was added to 500 µL of PBS, and 200 µL of the resulting solution was added to the circle of the PDMS sheet (diameter of 1 cm).
4. The dish was incubated at 37°C or at room temperature (RT) for 1 hour or more.

### <Day 0: Cell seeding to the dish>

### Preparations

TrypLE^{™} Select (containing 1 mM EDTA) (Thermo Fisher Scientific) was mixed with an equal amount of 0.5 mM EDTA/PBS to prepare 0.5 × TrypLE ^{™} Select (final concentration of 0.75 mM EDTA).

StemFit^{®} AK02N (Ajinomoto Co. Inc.) (a mixture of Solution A, Solution B, and Solution C of the product, hereinafter referred to as AK02N) was mixed well with 10 mM Y-27632 (TOCRIS) added to the medium in an amount of 1/1000 of the medium (final concentration of 10 µM) (hereinafter referred to as AK02N + Y).

### Procedures

1. KhES-1_Rx::Venus cells (KhES-1 cells obtained by knocking a GFP variant called Venus in the locus of a retinal marker Rx) that reached optimal confluence were collected from the medium and washed twice with 1 mL of PBS (-).
2. The cells were incubated at 37°C and 5% CO₂ with 0.5 × TrypLE^{™} Select added at 500 µL/well.
3. After the 0.5 × TrypLE^{™} Select was removed, PBS (-) was added to the well at 2 mL/well and the cells were peeled off by spraying PBS (-).
4. The cells were collected into a 15 mL Falcon tube and the cell number was counted.
5. The required number of cells (5 × 10⁵ cells per dish) was transferred to another tube and centrifuged at 1000 rpm at 20°C for 5 minutes.
6. After the supernatant was removed, the cells were suspended with AK02N + Y to provide a cell suspension of 5 × 10⁵ cells/200 µL AK02N + Y
7. The iMatrix coating solution within the PDMS sheet was removed with an aspirator and the cell suspension (5 × 10⁵ cells/200 µL AK02N + Y) was added to the circle and incubated at 37°C for 30 minutes.
8. The PDMS sheet was removed from the dish with sterilized tweezers.
9. The cells was cultured at 37°C and 5% CO₂ with 300 µL of AK002 N + Y added to the dish.

### <Day 1: Medium replacement with AK02 + PS>

### Procedures

1. Penicillin-Streptomycin (15070-062, Thermo Fisher Scientific) was added to AK02N and mixed well (the resulting medium contained no Y-27632, which is hereinafter referred to as AK02N + PS).
2. The culture medium was removed with an aspirator and 500 µL of AK02N + PS was added to the dish.

### <Day 2: Start of differentiation induction>

### Procedures

1. The cells were washed three times with 1 mL of gfCDM (10% KSR) (Table 1).
2. The whole medium was replaced with 1 mL of gfCDM (10% KSR).

**Table 1**

| gfCDM (growth factor-free, chemically defined medium) (10%KSR) | |
|---|---|
| IMDM (31980-030, Thermo Fisher Scientific) | 125 mL |
| Ham's F-12 Nutrient Mix, GlutaMAX^{™} Supplement (31765-092, Thermo Fisher Scientific) | 125 mL |
| Chemically Defined Lipid Concentrate (11905-031, Thermo Fisher Scientific) | 2.5 mL |
| BSA* (A3156, Sigma Aldrich) | 5 mL |
| 1-thioglycerol (207-09232, FUJIFILM Wako Pure Chemical Corporation) | 9.75 µL |
| Penicillin-Streptomycin (15070-062, Thermo Fisher Scientific) | 2.5 mL |
| KnockOut^{™}Serum Replacement (KSR) (10828028, Thermo Fisher Scientific) | 25 mL |
| Filtered | |

| | |
|---|---|
| *BSA (5g) was added to 20 mL of water for cell culture (W3500, Sigma Aldrich) and left for several hours to dissolve. | |

### <Day 3, Day 6: Addition of BMP inhibitor>

1. The whole medium was replaced with 1 mL of gfCDM (10% KSR) containing LDN-193189 (04-0074-02, ReproCELL Inc.) (hereinafter referred to as LDN). (final concentration of 100 nM LDN).

### <Day 8: Addition of BMP>

1. The cells were washed three times with 1 mL of gfCDM (10% KSR).
2. The whole medium was replaced with 1 mL of gfCDM (10% KSR) containing BMP4 (final concentration of 1.5 nM BMP4). The BMP4-containing medium was prepared by adding a 1 µM BMP4 solution, which was prepared by diluting10 µg of BMP4 (R&D Systems) with 275 µL of 0.1% BSA, to gfCDM (10% KSR).

### <Day 11, Day 14: Replacement of half of the medium>

1. After 500 µL of the medium was removed, 500 µL of gfCDM (10% KSR) was added to the dish (half replacement). Then, the half amount of the medium was replaced every 3 days.

### <Day 17: Replacement of the medium with DMEM/F-12 + N2>

1. The cells were washed three times with 1 mL of DMEM/F-12, GlutaMAX^{™} supplement (10565-042, Thermo Fisher Scientific).
2. One mL of DMEM/F-12 + N2 (Table 2) was added to the dish.

**Table 2**

| DMEM/F-12 + N2 | |
|---|---|
| DMEM/F-12, GlutaMAX^{™} supplement (10565-042) | 5 mL |
| N2 Supplement (17502-048 Thermo Fisher Scientific) | 50 µL |
| Penicillin-Streptomycin (15070-062) | 50 µL |
| Filtered | |

3. The cells were cultured to Day 20.

### <Day 20: Culture in the control medium for maturation>

1. The cells were washed three times with 1 mL of the control medium for maturation (Table 3).
2. The cells were cultured with 1 mL of the control medium for maturation added to the dish.

**Table 3**

| Control medium for maturation | |
|---|---|
| DMEM/F-12, GlutaMAX^{™} supplement (10565-042) | 500 mL |
| N2 Supplement (17502-048) | 5 (1 vial) |
| FBS (inactivated) (final concentration of 10%) (Sigma Aldrich) | 50 mL |
| 50 mM Taurine in PBS (final concentration of 100 µM) (T0625, Sigma Aldrich) | 1.12 mL |
| Antibiotic-Antimycotic (100X) (15240-062, Thermo Fisher Scientific) | 5 mL |
| Filtered | |

The medium was then replaced once every 2 to 3 days.

To prepare cells without patterning culture (non-patterned cells), cells were cultured according to the same procedure without the PDMS sheet in a microdish (µ-Dish 35 mm, High, 81156, ibidi) the entire surface of which was coated with iMatrix-511 (Nippi. Inc.) (0.25 µg/cm²).

### II. Effects of patterning culture on retinal differentiation

### II-1. Method for analysis

### - RNA sequencing (RNA-seq)

1. Total RNA was prepared with RNeasy Micro kit (QIAGEN) from the Day 8 sample of the patterned or non-patterned cells.
2. A sequence library was prepared by using Next Ultra II Directional RNA Library Prep Kit for illumine E7760 (NEB) and the sequences were determined by the next generation sequencer (NextSeq, illumina).
3. After the quality of the sequence data was checked by using FastQC (v0.11.9), TrimGalore (0.6.1), cutadapt (1.18), Bowtie2 (v2.4.19), the mapping was performed on STAR (2.7.5 a), samtools (1.1), SUBREAD (v2.0.1), and then the secondary analysis was performed with R (version 3.6.3), edgeR (v3.28.1).

### Gene ontology (GO) analysis

For the list of differentially expressed genes obtained by the secondary analysis of II-1, enrichment analysis of GO Biological processes was performed by using Metascape.

### Single cell RNA-seq (scRNA-seq)

1. The retinal tissue of Day 100 was dissociated into single cells by using TrypLE^{™} Select (Thermo Fisher Scientific) containing 150 µg/ml DNase I (Roche) at 37°C for 5 minutes and neutralized with 0.04% BSA/PBS.
2. The dissociated cells were pelleted, resuspended with 0.04% BSA/PBS, and passed through a 35 µm filter to provide a suspension of single cells.
3. A cDNA library was prepared from about 10,000 single cells by using Chromium Single Cell 3' Reagent Kit v3 (10x, Genomics). The sequences were determined by using a paired-end 100 cycle kit (28+8+91) with Illumina NovaSeq sequencer.
4. The mapping was performed by CellRanger (version 3.0.2) and the secondary analysis was performed by Seurrat (version 3.0).

The following markers were used for each type of cells:
Stalk cells (Stalk): VAX1/PAX2
Retinal pigment epithelial cells (RPE): MITF/PMEL
Amacrine cells (AC): ONECUT3/PRDM13
Retinal ganglion cells (RGC): POU4F2
Photoreceptor cells (PR): CRX/NEUROD4
Neural progenitor cells (NPC): HES6/NEUROD1
Retinal progenitor cells: CHX10/PAX6

### Observation of GFP expression

GFP-expressing cells were observed with Keyence all-in-one microscope BZ-X. After the phase difference image and GFP fluorescence image were obtained with a 10X lens, the entire image of the colony was created by tiling.

### Immunostaining

Expressions of Chx10, Recoverin, FOXG1, Crx, Tuj1, Zo-1, and Pax6 were evaluated by immunostaining with primary antibodies specific for the respective markers and secondary antibodies suitable for the primary antibodies.

### II-2. Induction of retinal differentiation in patterning culture

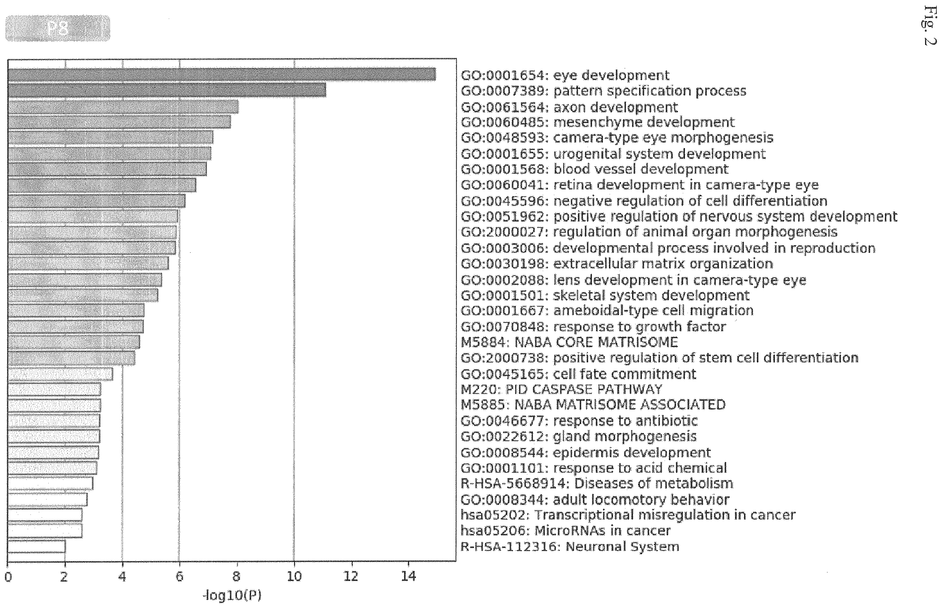

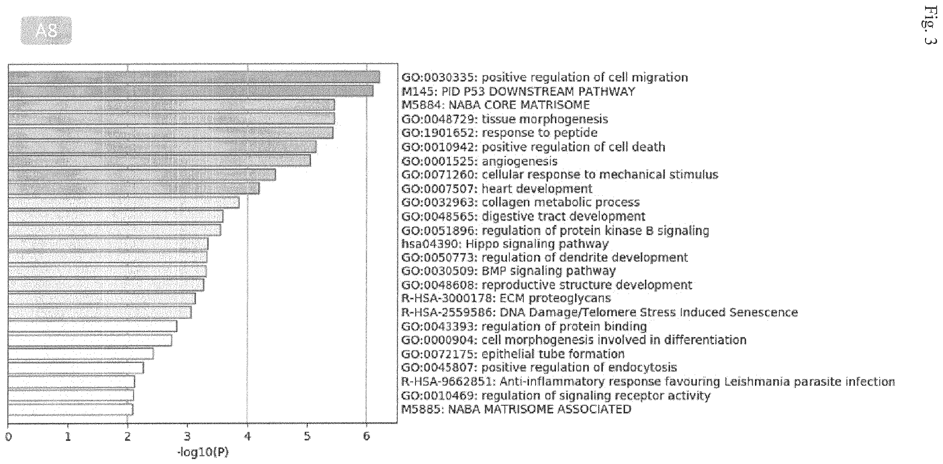

The gene expression of the patterned cells was compared with that of the non-patterned cells (seeded on the entire surface of the dish). RNA-seq and GO analysis were performed with the cells before the addition of BMP4 (Day 8). In the patterned cells, unlike the non-patterned cells, neural differentiation was enhanced prior to the addition of BMP4, which induced retinal differentiation (Fig. 2). In the non-patterned cells, BMP and Hippo signals were enhanced (Fig. 3).

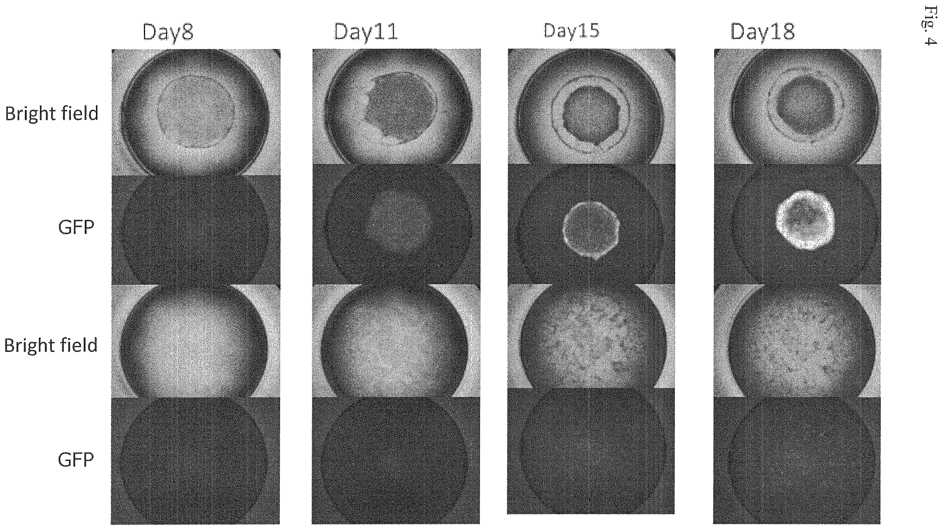

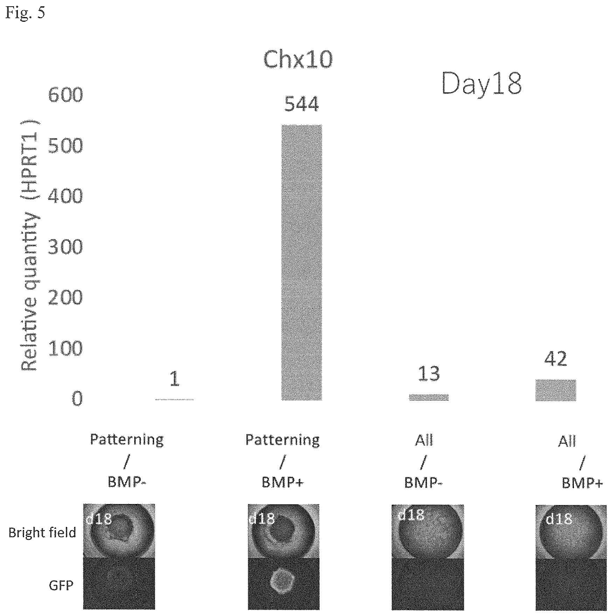

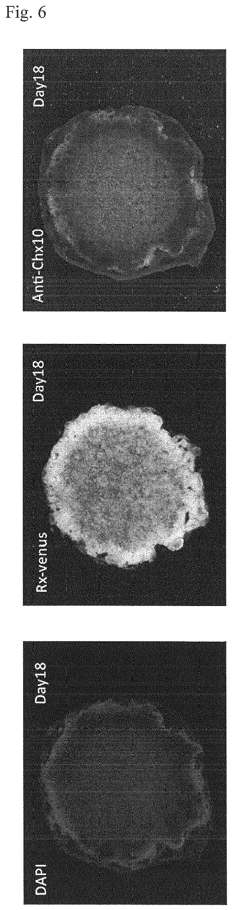

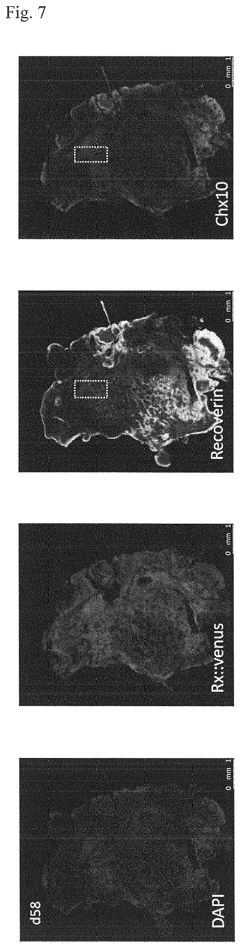

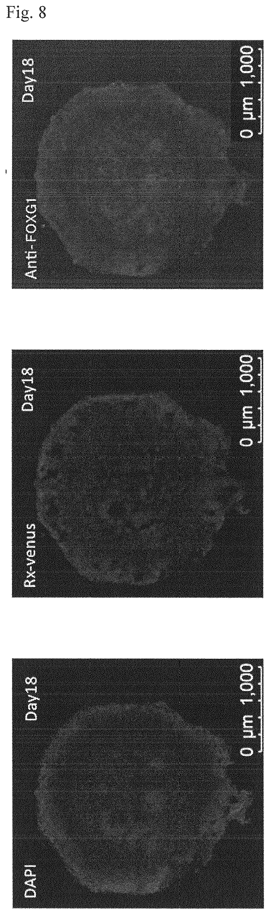

Retinal differentiation after the addition of BMP4 was evaluated by observing GFP (venus) expressed under the promoter of Rx, a retinal marker, over time. GFP expression was observed earlier in the patterned cells, indicating that the efficiency of differentiation induction of the cells was better than that of the non-patterned cells (Fig. 4). Also, on Day 18, expression of Chx10, a marker for bipolar cells and its progenitor cells, was evaluated. The expression with or without the BMP4 addition was evaluated for each of the patterned and non-patterned cells. The expression of Chx10 was assessed by quantitative PCR (using HPRT1 as an endogenous control) and immunostaining. As shown in Fig. 5, Chx10 expression was significantly increased in the patterned cells compared to the non-patterned cells. Fig. 6 shows a photograph of cells at Day 18 after the retinal differentiation by patterning culture. Rx and Chx10 were expressed in almost the entire region of the surface, suggesting that 95% or more of the cells expressed these markers. On Day 58, Recoverin, a marker of retinal photoreceptor cells, was expressed in the cells after the retinal differentiation with patterning culture (Fig. 7). In the cells after the patterning culture without the BMP4 addition, FOXG1, a marker of telencephalon, was expressed at Day 18 (Fig. 8)

### II-2. Differentiation efficiency

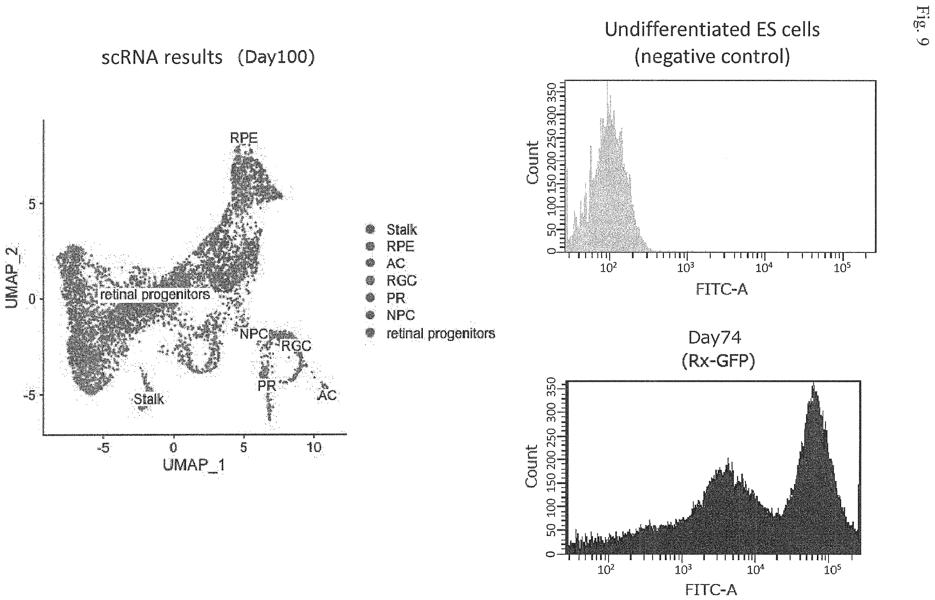

scRNA-seq was performed on the cells after the retinal differentiation with patterning culture at Day 100. scRNA-seq indicated that 97% or more of the cells were differentiated into retinal cells (Fig. 9, left). GFP expression at Day 74 of the cells was evaluated by FACS, and 90% or more of the cells were GFP-positive (Fig. 9, right).

### II-3. Size of patterning culture

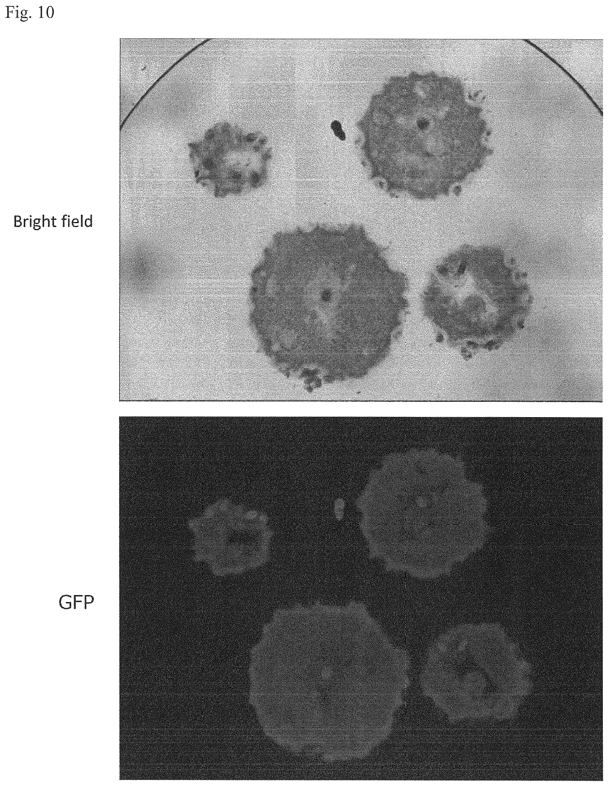

PDMS sheets each having a circular hole with a diameter of 2 mm, 5 mm, 7 mm, or 1 cm were prepared, and retinal differentiation was induced according to the same procedure. GFP expression at Day 20 was evaluated and differentiation induction was confirmed in any hole size (Fig. 10).

### II-4. Polarity of retinal tissue

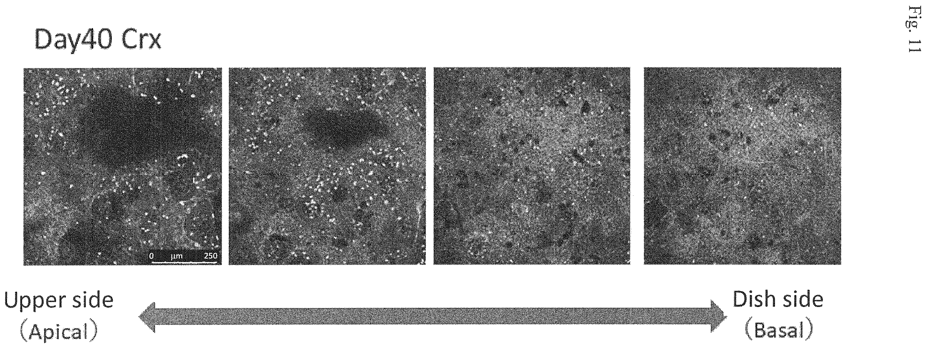

The polarity of the differentiated tissue was evaluated. In the retinal tissue of Day 40, expression of Crx, a marker of photoreceptor cells, was evaluated by immunostaining. Crx expression was observed at the top of the tissue (apical side), indicating that the differentiated tissue had the polarity of the retina (Fig. 11).

### II-5. Differentiation induction from other ES or iPS cell lines

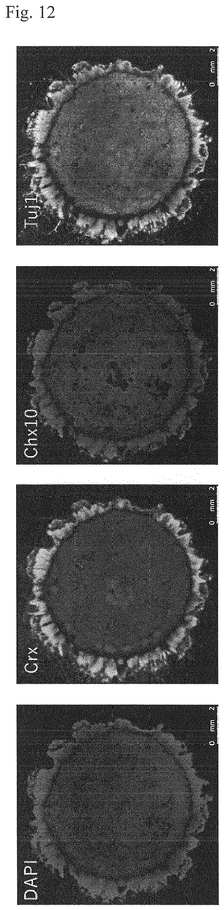

Retinal differentiation was induced by patterning culture according to the same procedure with KthES 11 cells, and expressions of Crx, Chx10, and a neural cell marker Tuj 1 at Day 50 were evaluated by immunostaining. These markers were expressed throughout the tissue, indicating that retinal differentiation was also induced in KthES 11 cells as in KhES-1_Rx: Venus cells (Fig. 12).

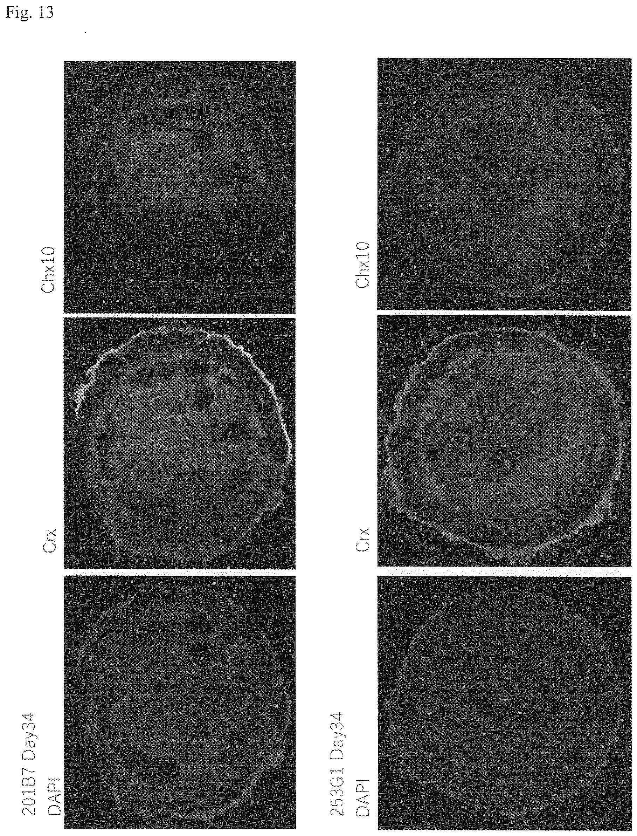

Similarly, iPS cell lines 201B7 cells and 253G1 cells were used to induce retinal differentiation and evaluated for Crx and Chx10 expressions at Day 34. Crx and Chx 10 were expressed throughout the tissue, indicating that retinal differentiation was also induced in these iPS cells (Fig. 13). Further, expressions of Zo-1 and Chx10 of the tissue derived from 201B7 cells at Day 41 were evaluated by immunostaining. Zo-1 is a tight junction marker. The expressions of Zo-1 and Chx10 were observed as layers, indicating that the differentiated tissue had a layered structure of the retina (data not shown).

### III. BMP signaling inhibitor

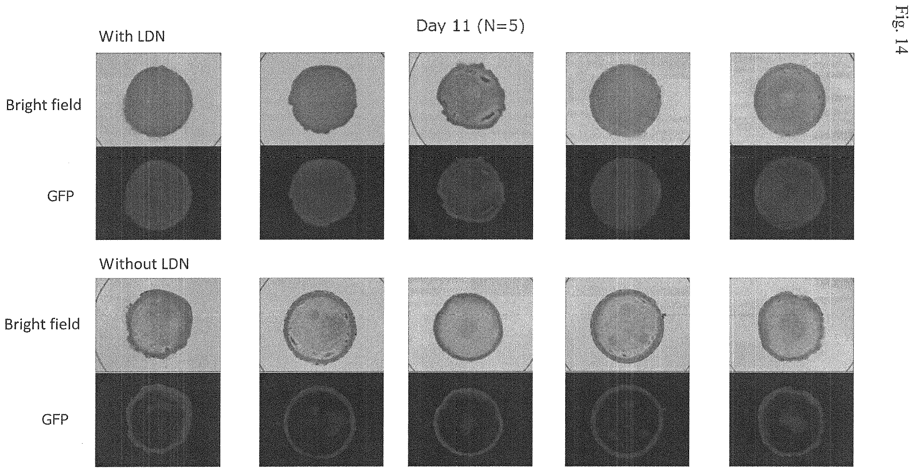

The effect of LDN-193189, a BMP signaling inhibitor, on the differentiation induction was evaluated. GFP expression at Day 11 was compared between cells prepared according to the patterning culture as described above, which comprised LDN-193189 addition from Day 3 to Day 8, and cells prepared according to the same procedure without the LDN-193189 addition. The cells with the LDN-193189 addition showed uniform GFP expression compared to the cells without the LDN-193189 addition (Fig. 14).

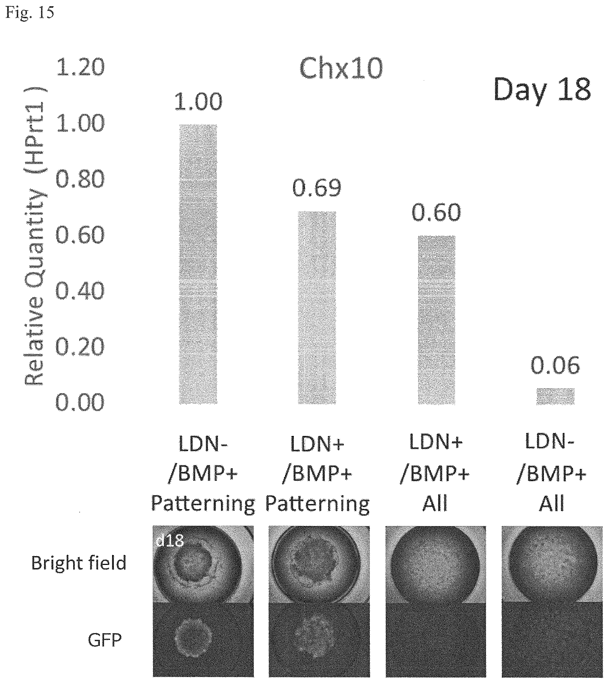

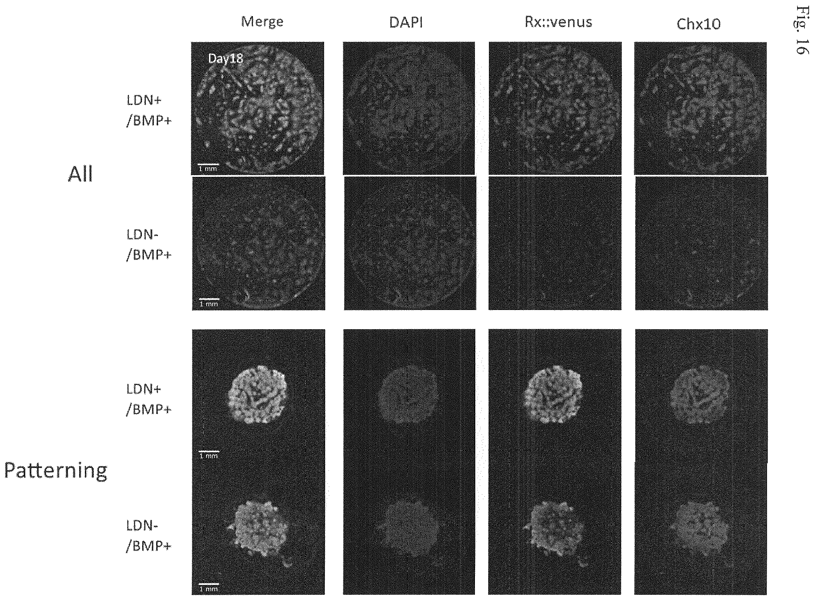

Also, expressions of GFP and Chx10 at Day 18 were evaluated in the patterned and non-patterned cells with or without the LDN-193189 addition. The Chx10 expression was assessed by quantitative PCR (using HPRT1 as an endogenous control) and immunostaining. Even without the patterning culture, the addition of LDN-193189 increased GFP and Chx10 expressions, indicating that induction to the retina was facilitated (Figs. 15 and 16).

### IV. Patterning culture with hydrophilic polymer

Patterning culture was performed with a non-cell-adhesive region prepared by coating with a MPC (2-methacryloyloxyethyl phosphorylcholine) polymer. A circular PDMS sheet having a diameter of 1 cm was put on a dish (µ-Dish 35 mm, High, 81156, ibidi). After the dish was coated with a solution containing 0.5 vol% MPC polymer in ethanol, the PDMS sheet was removed from the dish. To the dish, a solution prepared by adding 7 µL of iMatrix-511 (Nippi. Inc.) to 500 µL of PBS was added. According to these procedures, iMatrix did not adhere to the area pre-coated with the MPC polymer, and only the 1 cm circular area, which was protected with the PDMS sheet, was coated with iMatrix. Retinal differentiation was induced on this dish according to the same procedure except that Iwp2 (REPROCELL) (2 µM) was added from Day 20.

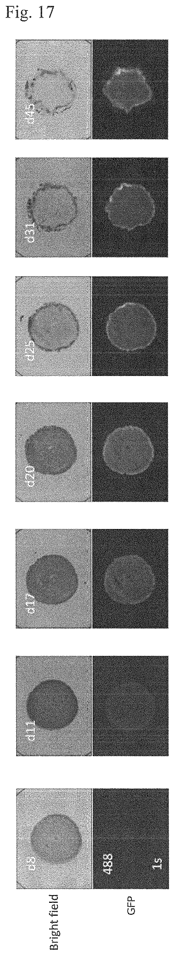

Even when a non-cell-adhesive region was prepared by MPC polymer coating, efficient and uniform GFP expression was observed (Fig. 17).

## Claims

1. A method of producing a retinal tissue, comprising inducing differentiation of pluripotent stem cells into a retinal tissue on a surface of a culture substrate, wherein the culture substrate comprises a region A and a region B on the surface, the differentiation is induced is the region A, the region A has cell adhesiveness, and the region B is adjacent to at least a part of the region A and has cell adhesiveness lower than the cell adhesiveness of the region A.

2. The method according to claim 1, wherein the region A is coated with a cell-adhesive material.

3. The method according to claim 2, wherein the cell-adhesive material is laminin.

4. The method according to any one of claims 1 to 3, wherein the region A is surrounded by the region B.

5. The method according to any one of claims 1 to 4, wherein the region B is coated with a non-cell-adhesive material.

6. The method according to claim 5, wherein the non-cell-adhesive material is an MPC polymer.

7. The method according to any one of claims 1 to 6, wherein the inducing differentiation of pluripotent stem cells into a retinal tissue comprises the following steps:
(1) culturing pluripotent stem cells in a medium containing a BMP signaling inhibitor; and
(2) culturing the cells obtained by the step (1) in a medium containing a BMP signaling activator.

8. The method according to claim 7, wherein the BMP signaling activator is BMP4.

9. The method according to claim 7 or 8, wherein the BMP signaling inhibitor is LDN-193189.

10. A method of producing a retinal tissue, comprising the following steps:
(1) culturing pluripotent stem cells in a medium containing a BMP signaling inhibitor; and
(2) culturing the cells obtained by the step (1) in a medium containing a BMP signaling activator.

11. A retinal tissue produced by the method according to any one of claims 1 to 10.

12. A sheet-like pluripotent stem cell-derived retinal tissue having a circular shape with a diameter of 1 to 10 cm.

13. A composition comprising the retinal tissue according to claim 11 or 12.

14. The composition according to claim 13, wherein the composition is for use in the treatment of a disease.

15. The composition according to claim 14, wherein the disease is retinitis pigmentosa.

16. The composition according to claim 13, wherein the composition is for use in evaluating toxicity or efficacy of an agent.
